# FASCICULE DE BREVET EUROPEEN

(11) **EP 0 633 745 B1**
(45) Date de publication et mention de la délivrance du brevet: **26.06.1996**
(21) Numéro de dépôt: 94905753.3
(22) Date de dépôt: 28.01.1994
(51) Int. Cl.: A61B 3/113, A61B 5/00

(54) **DISPOSITIF D'ANALYSE DU MOUVEMENT D'UN OEIL**
VORRICHTUNG ZUM ANALYSIEREN DER AUGENBEWEGUNG
DEVICE FOR EYE MOVEMENT ANALYSIS

(30) Priorité: 29.01.1993 FR 9301268
(43) Date de publication de la demande: 18.01.1995
(73) Titulaire: CENTRE NATIONAL DE LA RECHERCHE SCIENTIFIQUE (CNRS), 75794 Paris Cédex 16 (FR)
(72) Inventeur: GUILLEMANT, Philippe, F-13470 Carnoux (FR); ULMER, Erik, F-13009 Marseille (FR)
(74) Mandataire: Martin, Jean-Jacques
(86) Numéro de dépôt international: FR9400108
(87) Numéro de publication internationale: WO9416612

(56) Documents cités:
- EP-A- 0 125 808
- EP-A- 0 456 166
- GB-A- 2 207 748
- US-A- 4 852 988
- MEDICAL AND BIOLOGICAL ENGINEERING AND COMPUTING, vol.28, no.4, Juillet 1990, STEVENAGE GB pages 317 - 324 YAMADA ET AL. 'Development of an eye-movement analyser possessing functions for wireless transmission and autocalibration.'

## Description

La présente invention est relative à un dispositif d'analyse du mouvement de l'oeil ou des yeux d'un patient c'est-à-dire d'un être humain ou animal.

Le domaine technique de l'invention est celui de la fabrication de dispositif de mesure du mouvement des yeux.

L'intérêt de l'invention est avant tout d'ordre médical. Son application préférentielle est l'exploration des vertiges et des troubles de l'équilibre, qui sont l'une des principales pathologies auxquelles les médecins sont confrontés (un malade sur vingt en médecine générale).

Dans ce domaine d'application préférentielle de l'invention, l'oeil est considéré comme un témoin de l'activité du vestibule (ou labyrinthe postérieur), qui est un organe de l'équilibre situé dans l'oreille interne sensible à la position absolue ainsi qu'aux mouvements de la tête. Cet organe influence par voie réflexe (donc de façon involontaire) le tonus de tous les muscles squelettiques, en particulier celui des muscles oculaires. Lorsqu'il est stimulé, soit par des mouvements de la tête, soit de façon artificielle en particulier par des effets thermiques dans l'oreille interne, le vestibule est capable d'induire des mouvements automatiques des yeux ou nystagmus, qui sont synchrones pour les deux yeux. Par conséquent, l'observation de l'un quelconque de ces deux yeux (si la musculature oculaire est normale) est un excellent reflet du fontionnement du vestibule. Cependant, il faut tenir compte d'une contrainte importante qui résulte du fait que l'oeil répond aussi aux stimulations visuelles et que le système de la vision est prioritaire et plus puissant que celui du vestibule: l'observation du nystagmus ne témoigne donc du fonctionnement de l'oreille interne que si le patient est dans l'obscurité totale, c'est à dire si ses deux yeux ne recoivent aucune lumière visible.

Pour tester le vestibule à partir du témoin oculomoteur il faut donc réunir plusieurs conditions:
- les mouvements oculaires doivent pouvoir être observés et/ou analysés en permanence durant tous les tests de l'examen.
- l'examen doit pouvoir vérifier la normalité des mouvements oculaires volontaires et réflexes d'origine visuelle, durant des tests appelés "tests de poursuite", qui consistent à projeter des cibles ou stimuli visuels fixes ou mobiles proposés au regard du patient dans le décor.
- les stimuli vestibulaires naturels de déplacement auxquels est soumis le patient et pour lesquels on étudie la réponse oculomotrice (essentiellement des mouvements de sa tête par rapport au décor ou de sa tête par rapport à son thorax) doivent pouvoir être enregistrés et analysés en même temps que les mouvements de l'oeil témoin.
- l'examen doit pouvoir inclure des tests appelés "tests vestibulaires" durant lesquels est rendue nécessaire l'absence totale de lumière visible par le patient uniquement.

Pour mieux comprendre l'objectif de la présente invention, il faut aussi tenir compte du fait que la contrainte d'étanchéité à la lumière visible, si elle est indispensable à l'exploration de l'activité vestibulaire, peut devenir limitative dans le cas d'examens plus restreints pratiqués par d'autres médecins, pour lesquels l'observation et l'analyse des mouvements oculaires présente un intérêt légèrement différent, lorsque par exemple sont effectués uniquement des tests de poursuite ou encore de suivi et d'analyse du regard. Dans ce cas un dispositif selon l'application préférentielle pourrait être inadapté s'il ne prévoit pas dans sa réalisation que la solution apportée à la condition d'étanchéité ne soit mise en oeuvre que comme option secondaire, supplémentaire et indépendante du dispositif de base.

L'objectif principal de la présente invention est donc de procurer un dispositif et procédé d'observation, de mesure et d'analyse des mouvements des yeux, qui permette de mettre en oeuvre durant un même examen préférentiellement médical, les quatre priorités suivantes, exeptée la dernière pour certains examens, à savoir par ordre de priorité, premièrement la nécessité de pouvoir observer et/ou analyser le mouvement d'un oeil au moins durant la totalité de l'examen, deuxièmement la nécessité de pouvoir libérer le champ de vision d'un oeil au moins face au décor, troisièmement la nécessité de pouvoir observer et/ou analyser, en même temps que les mouvements d'un oeil au moins, tous les stimuli oculaires visuels et vestibulaires c'est à dire les mouvements des cibles visuelles par rapport à la tête, les mouvements de la tête par rapport au décor et les mouvements de la tête par rapport au thorax, quatrièmement la nécessité de pouvoir occulter totalement les deux yeux de toute lumière visible, c'est à dire de pouvoir placer le patient dans le noir complet durant une partie de l'examen.

Un autre objectif de l'invention est de procurer un dispositif qui soit ergonomique, susceptible de s'adapter à la morphologie et/ou à la vue du patient et d'être économique, compact et léger.

On connaît déjà par la demande de brevet EP 125 808 (WEINBLATT) un dispositif de mesure du mouvement des yeux qui comporte une monture de lunette et qui comporte, comme représenté à la figure 2 de ce document, une caméra de décor reliée à une optique fixée sur une branche de la lunette par des fibres optiques, et qui comporte également une caméra d'observation de l'oeil reliée à une optique fixée sur la monture de lunette, par l'intermédiaire de fibres optiques également; les signaux vidéo produits par les deux caméras sont combinés pour fournir une image correspondant à la superposition de l'image de décor avec l'image de l'oeil, lesquelles images sont observées par les deux caméras.

Un objectif de l'invention est de procurer un dispositif de mesure et d'analyse du mouvement des yeux comportant une caméra d'observation de l'oeil, et une caméra d'observation du décor dans lequel est placé le patient, qui soit amélioré par rapport à celui décrit dans le document ci-dessus, tout en restant compatible avec l'objectif principal. On connait également d'autres brevets ou publications sur des dispositifs de mesure du mouvement des yeux qui comportent une caméra de décor en relation avec une caméra d'observation de l'oeil, dans lesquels la caméra de décor est placée et équipée de façon à visualiser approximativement ou exactement le champ de vision de l'observateur. Ce dernier objectif n'est pas partagé en ce qui concerne la présente invention, pour laquelle la visualisation du décor a pour unique fonction l'observation et le calcul des mouvements relatifs d'objets du décor par rapport à la tête du patient (observateur), et pourrait en conséquence ne pas correspondre du tout au champ de vision de l'observateur (car rien n'empèche dans une configuration particulière du dispositif de placer ou projeter les objets du dispositif au plafond de la pièce d'examen et de diriger la caméra de décor vers le haut), ce qui a pour avantage de permettre à la présente invention une liberté de configuration du dispositif selon des critères qui sont adaptés à l'examen préférentiel et qui portent sur le positionnement de la caméra de décor par rapport à la caméra oculaire.

Les objectifs de l'invention sont atteints en procurant un dispositif d'analyse du mouvement de l'oeil comportant une première caméra vidéo, susceptible de délivrer un premier signal vidéo représentatif d'une image dudit oeil, lequel dispositif comporte en outre une deuxième caméra vidéo, susceptible de délivrer au moins un deuxième signal vidéo représentatif d'une image d'un décor, lesquelles première caméra et deuxième caméra sont montées sur une monture de lunette, dans lequel lesdites première caméra et deuxième caméra sont mécaniquement rigidement liées entre elles et font partie d'un module amovible, et dans lequel lesdites première caméra et deuxième caméra ont des points de visée de leurs scènes respectives qui sont rapprochés.

L'invention procure un dispositif de mesure et d'analyse du mouvement des yeux comportant une caméra d'observation de l'oeil, et une caméra d'observation du décor dans lequel est placé le patient, qui exploite une configuration de la position de ces deux caméras (l'une par rapport à l'autre) qui est à la fois la plus pratique mécaniquement et optimale de sorte que les calculs des mouvements relatifs des objets des images de l'oeil et du décor par rapport aux deux caméras sont simplifiés, plus rapides et plus précis, de sorte que les composantes de ces mouvements sont plus faciles à corréler entre elles, et les réglages et l'étalonnage préalables nécessaires avant d'effectuer les mesures sont facilités.

Selon un mode préférentiel de réalisation, l'invention procure un dispositif de mesure et/ou d'analyse du mouvement de l'oeil et/ou des mouvements des yeux d'un patient, c'est-à-dire d'un être humain ou animal, comportant au moins une première caméra vidéo, sensible à la lumière infrarouge et particulièrement à la lumière du domaine du proche infrarouge, et de préférence sensible à une partie au moins de la lumière visible, laquelle première caméra vidéo est susceptible de délivrer au moins un premier signal vidéo représentatif d'une image dudit oeil ; ledit dispositif comporte en outre au moins une deuxième caméra vidéo, sensible à la lumière infrarouge et particulièrement à la lumière du domaine du proche infrarouge, de préférence sensible à une partie au moins de la lumière visible, laquelle deuxième caméra vidéo est susceptible de délivrer au moins un deuxième signal vidéo représentatif d'une image d'un décor, tel qu'un objet au moins et/ou une scène et/ou un tableau et/ou un écran sur lequel une image peut être projetée, lequel décor est proche et/ou situé à proximité dudit patient ; lesdites première caméra et deuxième caméras sont montées sur un masque ou monture de lunette, susceptible d'être portée par ledit patient, lesdites premières et deuxièmes caméras sont mécaniquement sensiblement rigidement liées entre elles ; ledit dispositif comporte un module facilement amovible ou interchangeable par emboîtage ou enclipsage par exemple sur ledit masque, lequel module comporte lesdites première et deuxième caméras vidéo, et lesdites première et deuxième caméras ont des points de visée de leurs scènes respectives (c'est-à-dire par exemple leur foyer optique ou leur plan focal image) qui sont rapprochés et de préférence sensiblement confondus, et ont des axes de leurs champs de vision respectifs, correspondant aux lignes ou aux colonnes de leurs deux images, qui ont au moins une direction commune.

Avantageusement, lesdites première et deuxième caméras ont des axes optiques respectifs sensiblement parallèles entre eux et proches, de préférence sensiblement confondus lesquelles première et deuxièmes caméras sont montées l'une par rapport à l'autre dans une configuration "tête bêche" ou "dos à dos" c'est-à-dire symétriquement par rapport à un plan médian, qui est sensiblement perpendiculaire à la direction selon laquelle ledit patient regarde ledit décor, c'est-à-dire que ledit plan de symétrie est sensiblement vertical et perpendiculaire à l'axe de visée ou d'observation du patient.

Avantageusement, ledit dispositif comporte des moyens de numérisation dudit deuxième signal vidéo, ledit dispositif comporte des moyens d'analyse et/ou de traitement des données numériques correspondant audit deuxième signal numérisé et/ou auxdites images de décor numérisées obtenues, lesquels moyens d'analyse comportent des moyens d'extraction de contour, comportent de préférence des moyens d'identification d'au moins un objet apparaissant dans ladite image de décor et comportent des moyens de calcul de coordonnées dudit objet.

Avantageusement, le dispositif comporte des moyens de numérisation du premier signal vidéo et des moyens d'analyse des images numérisées de l'oeil obtenues, lesquels moyens d'analyse et/ou de traitement des données numériques correspondant au premier signal vidéo numérisé comportent des moyens spécialisés de détection de la pupille et des moyens d'extraction de contours d'objets, des moyens d'identification de ces objets et des moyens de calcul des coordonnées du centre de la pupille et des autres objets extraits de l'image de l'oeil.

Avantageusement, ledit module, comportant lesdites première et deuxième caméras vidéo, comporte un premier capteur CCD matriciel monochrome et un deuxième capteur CCD matriciel monochrome, lesquels premier et deuxième capteurs CCD sont de préférence identiques et sont situés dans deux plans sensiblement parallèles entre eux, perpendiculaires auxdits axes optiques sensiblement confondus desdites caméras, et symétriques par rapport audit plan de symétrie, lesquels plans et/ou lesquels capteurs CCD sont très proches l'un de l'autre par exemple situés à une distance, mesurée sur l'axe optique commun auxdites caméras, qui est de l'ordre de 1 à 50 mm de préférence à une distance voisine de ou inférieure à 10 mm.

Avantageusement, ledit dispositif comporte des moyens de synchronisation desdits premier et deuxième signaux vidéo et des moyens de multiplexage desdits premier et deuxième signaux vidéo pour former un premier signal vidéo multiplexé composé de deux trames paires et impaires, dont l'une (paire ou impaire) est identique à la trame simultanée dudit premier signal vidéo et dont la deuxième (respectivement impaire ou paire) est identique à la trame simultanée dudit deuxième signal vidéo.

Avantageusement, ledit dispositif comporte des moyens de synchronisation desdits premier et deuxième signaux vidéo et des moyens de multiplexage desdits premier et deuxième signaux vidéo pour former un deuxième signal vidéo multiplexé lequel deuxième signal vidéo multiplexé est composé de deux trames paires et impaires, qui comportent chacune un ensemble consécutif de lignes qui sont, de préférence alternativement, identiques à des lignes desdits premier et deuxième signaux vidéo issus respectivement desdites première et deuxième caméras vidéo.

Avantageusement, le dispositif comporte un masque ou monture de lunette qui peut être muni par emboitage ou enclipsage de deux modules choisis parmi un ensemble de modules équipant le dispositif, lequel ensemble de modules comporte au moins deux modules amovibles, lesquels modules étant d'une part le module principal composé d'une caméra d'observation de l'oeil et d'une caméra d'observation du décor, et d'autre part un module d'observation et/ou d'analyse de l'oeil comportant une seule caméra, lesquels modules comportant tous les deux des moyens tels que des diodes, d'éclairage en lumière infrarouge de l'oeil.

Avantageusement, le dispositif comporte un masque ou monture de lunette muni de moyens d'étanchéité à la lumière de façon à ce que l'emboitage ou l'enclipsage de deux modules sur ledit masque permette de former devant lesdits yeux du patient une chambre noire ou un volume étanche à la lumière visible extérieure, et avantageusement l'ensemble de modules du dispositif comporte un module particulier qui comporte un cache vu obturateur ou bouchon ne possédant aucune caméra. Par ailleurs, le retrait de l'un de ces modules de ce masque permet de dégager avantageusement devant l'un des yeux du patient une ouverture lui permettant de voir le décor sous un angle suffisant (+/- 25° soit 50° au moins au total).

Avantageusement, l'un des modules du dispositif comporte pour certains examens exigeant une haute fréquence d'acquisition des mouvements oculaires une caméra d'observation et/ou d'analyse de l'oeil, et des moyens tels que des diodes d'éclairage en lumière infrarouge de l'oeil, et des moyens tels que des photorécepteurs de l'intensité lumineuse réfléchie par le globe oculaire.

Avantageusement, ledit décor ou environnement comporte au moins un repère ou objet contrasté fixe ou mobile, et de préférence ledit dispositif comporte des moyens de projection d'au moins une tâche lumineuse, de lumière visible ou infrarouge sur ledit environnement ou décor, laquelle tâche lumineuse constitue l'un desdits repères contrastés fixes ou mobiles, lesquels moyens de projection sont de préférence mécaniquement liés au corps dudit patient, de préférence au buste dudit patient.

Avantageusement, ledit dispositif comporte des deuxièmes moyens d'éclairage dudit décor et/ou de projection d'un repère dans ledit décor, lesquels moyens d'éclairage sont de préférence liés mécaniquement audit module amovible.

Les dispositifs de mesure selon l'invention procurent de nombreux avantages.

En effet grâce à la configuration modulaire du dispositif rendant indépendants le module d'observation oculaire et le masque ayant pour fonction la fixation de ce module sur la tête, le masque peut être librement choisi étanche ou non étanche à la lumière visible suivant les besoins de l'examen. Grâce à ce choix rendu possible, on peut utiliser pour certains examens une monture de lunette réduite à sa plus simple expression c'est à dire constituée par exemple uniquement d'un seul oculaire dans lequel un module vient s'emboiter ou s'enclipser, ce qui permet de libérer complètement le champ de vision d'un oeil au moins, fournissant ainsi un plus grand avantage pour l'étude de la position du regard. Par ailleurs grâce aux deux ouvertures pratiquées sur un masque étanche et aux écrans amovibles constitués par différents modules, le dispositif se prête aussi bien à des examens en présence de lumière visible pour lesquels un champ de vision suffisant subsiste à travers une ouverture, qu'à des examens dans lesquels une chambre "noire" étanche à la lumière visible extérieure, doit être prévue devant les yeux du patient.

Grâce au module principal du dispositif de caractéristique amovible et facilement tenu en main, ce module peut être utilisé avant d'être positionné sur le masque porté par le patient en étant simplement tenu par le bout des doigts d'un opérateur ou médecin, pour indiquer au système d'analyse un ou plusieurs objets particuliers du décor à identifier, en orientant par exemple le module de façon à placer ces objets un par un approximativement au centre de l'image délivrée par la caméra de décor, cette identification initiale étant utile pour mémoriser les caractéristiques de ces objets et permettre ultérieurement de quantifier les mouvements relatifs du module (et donc les mouvements de la tête du patient) à partir de l'identification à postériori de la position de ces mêmes objets déplacés dans l'image. En effet il peut être difficile de faire exécuter au patient lui même cette opération en bougeant sa tête lorsque le module est placé sur son masque, puisque le patient ne voit pas nécessairement l'écran de visualisation sur lequel apparait l'image délivrée par la caméra de décor.

Grâce à la configuration selon laquelle les deux caméras du module principal ont des points de visée de leurs scènes respectives qui sont rapprochés et de préférence confondus, on élimine en l'annulant ou en le négligeant un paramètre génant car non angulaire de distance entre les axes optiques qui intervient dans la transformation des coordonnées angulaires apparentes d'objets du repère d'une caméra à celui d'une autre et qui oblige à estimer, pour faire les corrections angulaires nécessaires, les distances des caméras aux objets utilisés pour le calcul de leurs mouvements. Grâce à ladite configuration la connaissance et l'usage embarrassant de ces distances très fluctuantes est rendue inutile, ce qui est en particulier et avantageusement le cas de la configuration "tête bêche".

Grâce à la configuration selon laquelle les deux caméras ont des axes de leurs champs de vision respectifs, correspondant aux lignes ou aux colonnes de leurs deux images, qui partagent au moins une direction commune, les mouvements purement horizontaux de l'oeil tête droite (ou les mouvements horizontaux du masque par rapport à la tête) peuvent correspondrent à des mouvements purement horizontaux des objets fixes du décor, et les mouvements purement verticaux de l'oeil tête droite (ou les mouvements verticaux du masque par rapport à la tête) peuvent correspondrent à des mouvements purement verticaux des objets fixes du décor, ce qui permet aux composantes de ces mouvements calculées par traitement électronique d'images d'être lors d'un examen habituel immédiatement disponibles sans étalonnage pour l'analyse et la corrélation des mouvements et ce qui simplifie les calculs qui sont aussi rendus plus rapides et plus faciles à effectuer en temps réel sur un simple micro-ordinateur.

Grâce à la configuration selon laquelle les deux caméras sont assemblées à l'intérieur d'un module ou boitier amovible et de préférence lorsque les deux caméras sont montées "tête bêche", l'encombrement des deux caméras est réduit au minimum et la présence de la caméra de décor n'agrandit pas la partie du champ de vision du patient déjà occultée par la première caméra d'observation de l'oeil; dans le cas d'utilisation d'une caméra d'observation de l'oeil et d'une caméra d'observation du décor et/ou des capteurs CCD correspondants identiques, lesdites caméras et lesdits capteurs CCD sont plus faciles à loger dans un même boîtier qui peut contenir les moyens électroniques de prétraitement des signaux issus des capteurs CCD et faciliter la liaison entre le module fixé de façon amovible à la monture de lunettes et le dispositif de traitement de signaux qui peut être incorporé dans un micro-ordinateur.

Grâce à la configuration modulaire et de préférence "tête bêche" permettant de rapprocher au mieux les deux caméras à l'intérieur d'un boitier ou module principal, selon laquelle ledit boîtier ou module fixé de façon amovible sur la monture de lunette comporte les moyens électroniques de commande et de synchronisation des mesures des capteurs CCD, on peut éviter les phénomènes de parasites électromagnétiques pouvant interférer sur les signaux de mesure; enfin ledit boîtier ou module amovible peut contenir l'électronique d'alimentation des diodes d'éclairage de l'oeil en lumière infrarouge et de réception de signaux délivrés par des photorécepteurs, dans le cas d'utilisation de tels photorécepteurs qui sont intégrés audit module et qui permettent d'observer au moins deux composantes de mouvement de l'oeil, avec une bande passante beaucoup plus élevée que la bande passante vidéo standard qui est de l'ordre de 25 hertz.

Grâce à la configuration desdites caméras et/ou desdits capteurs CCD dont les axes optiques sont confondus et qui sont montés "tête bêche", les images délivrées par lesdites caméras sont plus simples à corréler et le calcul des mouvements des objets du décor notamment par rapport à la position des yeux est plus précis; grâce à la position des caméras dont les axes optiques sont confondus, l'étalonnage du dispositif est simplifié; en effet dans le cas d'un retrait temporaire possible du masque ou de la monture de lunette par le patient ou d'un simple réajustement de ladite monture par rapport à la tête du patient, ledit réajustement provoque une indétermination sur la position des caméras par rapport à la tête, qui peut engendrer des erreurs dans les mouvements calculés à partir des images desdites caméras. Grâce à la correction des erreurs sur les mouvements oculaires qui peut être faite au moins en partie en utilisant comme repère de référence des zones naturellement contrastées du visage qui sont fixes par rapport à la tête, et grâce à ladite configuration "tête bêche", les erreurs sur les mouvements des objets du décor peuvent aussi être corrigées au moins en partie en se servant du même repère, ce qui peut permettre d'éviter un nouvel étalonnage du fait de ce réajustement du masque.

Grâce à la configuration du dispositif préférentiel utilisé avec un masque étanche, pour lequel existent sur ce masque deux ouvertures oculaires obturables par deux modules amovibles distincts, on peut observer et/ou analyser chaque oeil avec un module différent donc éventuellement avec des procédés d'analyse différents, des capteurs différents, des éclairages différents, et pratiquer ainsi simultanément sur deux yeux deux analyses ou tests différents et impossibles à pratiquer simultanément sur le même oeil. Par exemple, en cas de difficulté pour positionner sur une petite surface du même module, diodes, photorécepteurs et objectif nécessaires à une analyse particulièrement exigeante (comme celle des saccades de l'oeil qui nécessite une bande passante élevée de l'ordre de 250Hz), on peut utiliser un module droit équipé de diodes et d'objectif, et un module gauche équipé de diodes et de photorécepteurs. Cette spécialisation ou différenciation des modules est par ailleurs nécessaire compte tenu du fait qu'il est inutile donc onéreux d'utiliser deux caméras de décor, donc il est avantageux d'observer et/ou d'analyser les deux yeux simultanément dans le noir grâce à un module équipé d'une seule caméra de type oculaire, accompagnant le module principal équipé de deux caméras. Il est également avantageux de pouvoir utiliser un module réduit à sa plus simple fonction d'écran ou de "bouchon" pour pratiquer des tests monoculaires généralement suffisants et allégeant ainsi le dispositif.

Grâce à la configuration du dispositif comportant des moyens dirigés sur un seul oeil ou sur les deux yeux d'analyse des mouvements oculaires synchrones par caméra avec simultanément des moyens d'analyse des mouvements oculaires par capteurs d'intensité lumineuse réfléchie par le globe oculaire, tels que des photorécepteurs, on dispose de deux bandes passantes complémentaires d'analyse de ces mouvements, respectivement de basse fréquence et de haute fréquence, pouvant être avantageusement combinées par une opération de traitement du signal revenant à une addition pour chaque axe horizontal ou vertical d'une part des déplacements oculaires enregistrés par photorécepteurs et sortant d'un filtre passe haut (>25 Hz) et d'autre part des déplacements oculaires calculés par traitement d'image et sortant d'un filtre passe-bas (< 25 Hz), de façon à former une réponse oculaire haute fréquence recalée sans étalonnage (ou avec étalonnage automatique) sur les mouvements exacts de l'oeil, ce qui évite très avantageusement d'avoir à utiliser des fréquences d'acquisition et de traitement d'images supérieures à 25 Hz.

Par ailleurs des photorécepteurs peuvent avantageusement être aussi placés sur l'autre face d'un module pour capter l'intensité lumineuse issue du décor et augmenter selon le même principe la fréquence de mesure des mouvements relatifs de la tête par rapport aux cibles ou repères lumineux.

Grâce aux moyens d'analyse et/ou de traitement des données numériques de l'image du décor numérisée obtenue, et en particulier grâce aux moyens d'extraction de contours dont est muni le dispositif, les contours des objets fixes naturellement présents ou artificiellement placés (pour les besoins propres de l'examen) dans le décor peuvent être calculés traités et reconnus individuellement de manière à suivre chaque objet dans son mouvement pour en déduire au moins les coordonnées apparentes horizontales, verticales et rotatoires du mouvement de la tête par rapport au décor.

Grâce aux mêmes moyens d'analyse et/ou de traitement des données et d'extraction de contour de l'image d'un oeil obtenue, les contours de la pupille et des reflets des diodes sur la cornée, ainsi que d'autres objets de l'image oculaire peuvent être identifiés et permettre en particulier le calcul des coordonnées du centre de la pupille.

Par ailleurs, dans le mode de réalisation où des moyens d'éclairage liés au buste du patient sont prévus qui projetent un repère dans le décor, la position du repère correspondant peut être déterminée par les même moyens d'analyse de l'image délivrée par la caméra de décor et permettre ainsi de déterminer par suivi des coordonnées du repère mobile projeté les mouvements du buste ou du corps du patient par rapport au décor.

Grâce à l'invention, il est donc possible d'analyser en même temps que les mouvements oculaires non seulement les stimulis labyrinthiques et proprioceptifs cervicaux qui sont à l'origine de ces mouvements oculaires et qui nécessitent la mesure des mouvements relatifs entre la tête et le décor et/ou entre la tête et le buste du patient, mais aussi les stimuli visuels qui peuvent être obtenus par les mêmes résultats de l' analyse d' images de décor fournissant les coordonnées des objets constitués par les cibles mobiles visuelles proposées à la vue du patient, qui sont distinctes des cibles mobiles éventuellement infrarouges proposées à la caméra de décor et non au patient.

Grâce à l'invention, on peut ainsi éviter par une seule analyse de l'image de décor l'utilisation de très nombreux capteurs ou dispositifs de stimulation qui sans l'invention sont indispensables, comme par exemple l'utilisation d'un fauteuil électroniquement asservi avec l'acquisition du mouvement de ce fauteuil sur lequel est assis le patient, l'acquisition des mouvements de sa tête (accéléromètres et/ou magnétomètres), ou l' acquisition du mouvement de moteurs de mise en rotation de miroirs de projection de rayon lumineux.

Grâce au multiplexage des images de l'oeil et du décor dans lequel ces images se partagent alternativement des ensembles consécutifs de lignes d'une même image (par exemple l'oeil en haut de l'image et le décor en bas), le médecin peut enregistrer sur magnétoscope une trace qualitative des stimulis visuels ou des mouvements de la tête du patient en même temps que ses mouvements oculaires ce qui peut lui permettre une démonstration ou un diagnostic qualitatif rapide en temps différé.

Grâce au multiplexage des images de l'oeil et du décor pour lequel ces images se partagent alternativement les deux trames d'une même image, le dispositif de traitement des images peut travailler sur un signal vidéo unique, ce qui simplifie l'électronique d'acquisition des images, réduit le coût électronique du dispositif, et permet d'entrer l'image complète de l'oeil et de la tête en même temps que l'image complète du décor, sur magnétoscope comme sur micro-ordinateur.

Grâce à des moyens d'éclairage infrarouge du décor liés mécaniquement au module amovible on peut utiliser comme objets fixes dans une pièce sombre pour le calcul des mouvements tête / décor des objets invisibles pour le patient comme par exemple des formes naturellement présentes ou des pastilles volontairement placées sur les murs sombres ayant la particularité de réfléchir plus ou moins cet éclairage infrarouge tout en restant bien sur invisibles à la vue du patient.

Grâce au procédé d'analyse du décor du dispositif consistant à calculer le mouvement apparent du décor par rapport à la tête en identifiant les positions successives du même objet dans l'image de décor, et grâce au procédé de calcul suivant de ce mouvement, lorsque la dynamique angulaire est supérieure à l'angle de l'objectif de la caméra de décor, consistant à relayer l'identification d'un objet de référence sortant de ce champ de vision par l'identification d'un autre objet de référence apparaissant dans ce champ de vision et donc mieux placé, le dispositif est capable de simuler les phases lentes et les saccades d'un mouvement de nystagmus correct que pourrait ou devrait produire les yeux du patient, en se servant éventuellement avantageusement du résultat du calcul de la position de son regard pour aider à identifier un nouvel objet de l'image du décor à poursuivre pour le calcul de son mouvement apparent.

Dans un mode préférentiel de mise en oeuvre d'un dispositif selon l'invention, la mesure des mouvements oculaires est effectuée de la façon suivante:
- dans une fenêtre de l'image centrée sur la position moyenne de la pupille et délivrée par ladite première caméra ou caméra d'observation de l'oeil, on isole le contour d'une forme correspondant à la pupille,
- on rectifie s'il y a lieu le contour de la pupille de façon à le rendre sensiblement circulaire,
- on calcule le barycentre géométrique du contour, qui est la position du centre de la pupille, c'est-à-dire ses deux coordonnées dans un plan,
- on compare une portion angulaire d'une couronne située autour de l'image de ladite pupille (comportant de façon connue des stries) que l'on compare ou corrèle avec une image préalablement enregistrée de ladite couronne angulaire, et on déduit un angle de rotation de ladite couronne angulaire correspondant à un angle de rotation dans ledit plan dudit oeil.

Dans un mode préférentiel de réalisation du procédé d'analyse du mouvement de la tête du patient, on effectue les opérations suivantes:
- on utilise une fenêtre ou une partie de l'image de décor fournie par la deuxième caméra, ladite fenêtre pouvant dans un mode particulier de mise en oeuvre se déplacer dans ladite image,
- on extrait les contours de différents objets détectés dans ladite fenêtre, et on calcule les barycentres des objets et/ou des contours correspondants,
- on élimine les contours ainsi détectés qui n'ont pas un nombre de points compris dans un intervalle centré autour du nombre de points préalablement enregistré d'un objet prédéterminé et recherché,
- on calcule, pour chaque contour subsistant, des fonctions réduites de forme de contour prédéterminées,
- on compare lesdites fonctions réduites de forme de contour ainsi déterminées avec des fonctions de forme de contour préalablement enregistrées et correspondant à l'objet recherché,
- on calcule la position dans ladite fenêtre dudit objet ainsi identifié, et on recentre dans un mode particulier de mise en oeuvre ladite fenêtre autour dudit objet.

Avantageusement, il est possible de visualiser simultanément en temps "quasi réel", c'est-à-dire à une fréquence de l'ordre de 25 hertz, une image d'une fenêtre ou partie de l'image de l'oeil et une image d'une fenêtre ou partie de l'image de décor, des courbes de variation en fonction du temps de la position du centre de la pupille déterminée comme indiqué ci-dessus, et éventuellement une courbe de la rotation de ladite pupille en fonction du temps, et des courbes de la position dudit objet identifié correspondant aux mouvements apparents de la tête.

Avantageusement, il est possible de visualiser en temps "quasi-réel" pour le contrôle visuel des calculs, une croix indiquant le centre calculé de la pupille sur l'image de l'oeil, laquelle croix pouvant être éventuellement inclinée pour refléter le résultat du calcul de la rotation de la pupille.

Les nombreux avantages procurés par l'invention, seront mieux compris au travers de la description suivante qui se réfère aux dessins annexés, qui illustrent sans aucun caractère limitatif, des modes particuliers de réalisation de dispositif selon l'invention.

La figure 1 illustre shématiquement en vue transversale un dispositif selon l'invention placé sur la tête d'un patient observant un décor.

La figure 2 illustre shématiquement des moyens de multiplexage des signaux vidéo délivrés par les caméras d'observation de l'oeil et du décor d'un dispositif selon l'invention.

La figure 3 illustre la face d'un module amovible d'un dispositif selon l'invention, qui est située du coté de l'oeil du patient, et qui correspond à un mode particulier de réalisation comportant des photo-diodes ou photo-récepteurs lumineux dont la bande passante peut être supérieure à 250 hertz.

La figure 4 illustre schématiquement des deuxièmes moyens de synchronisation des signaux vidéo issus des caméras d'observation de l'oeil et du décor d'un dispositif selon l'invention.

La figure 5 illustre schématiquement selon une vue en coupe transversale dans un plan sensiblement vertical d'un module amovible d'un dispositif selon l'invention.

La figure 6 illustre schématiquement un décor et les repères dont il est muni.

La figure 7 illustre un mode de visualisation des résultats de mesure des mouvements de l'oeil utilisant un dispositif selon l'invention.

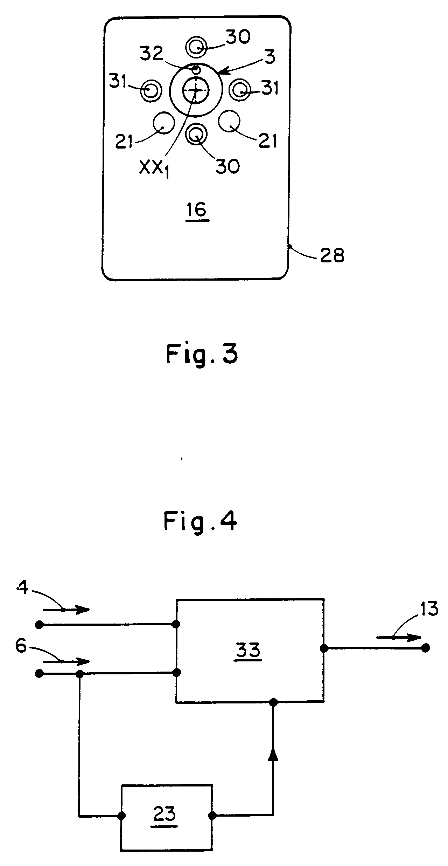

Le dispositif comporte un module 16 amovible comportant un boitier (repère 28 de la figure 3) qui reçoit une caméra 3 vidéo d'observation de l'oeil 2 d'un patient, et une caméra 5 vidéo d'observation du décor 7 observé par le patient dont le regard est pointé sensiblement selon la direction représentée par la flèche 29.

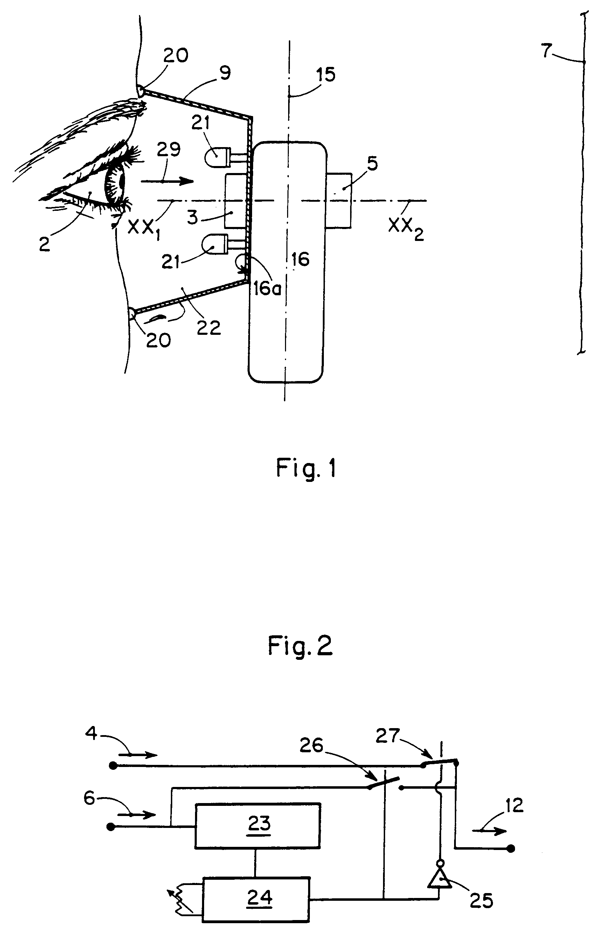

Ledit boitier dudit module amovible peut avantageusement contenir des moyens de prétraitement des signaux vidéo délivrés par lesdites caméras vidéo 3, 5, notamment ceux décrits aux figures 2 et 4.

Ledit module ou boitier 16 peut être monté et démonté manuellement et aisément sur un masque 9 porté par le patient et ayant la forme générale d'une monture de lunette.

Ledit module 16 peut être monté de manière étanche de façon à obturer par sa face 16a une ouverture prévue dans la partie frontale dudit masque ou de ladite monture de lunette ; ledit masque peut par exemple comporter deux ouvertures respectivement situées en regard des yeux du patient dont l'une peut être équipée de l'un desdits modules et dont la deuxième ouverture peut être par exemple équipée d'un cache obturant également de manière étanche à la lumière visible extérieure le volume interne 22 situé à proximité des yeux du patient et délimité par ledit masque, de manière à constituer devant les yeux du patient une chambre noire, c'est-à-dire une chambre isolée de la lumière visible extérieure audit masque, et ceci par exemple pour l'exécution de tests vestibulaires.

A cet effet, ledit masque peut être muni sur sa face venant en contact avec la tête du patient, de moyens d'étanchéité 20 permettant d'assurer l'obscurité dans ladite chambre 22.

Ledit module 16 est muni à cet effet, sur sa face interne, de diodes électroluminescentes 21 émettant de la lumière dans le domaine infrarouge ou proche infrarouge, ceci afin d'éclairer les yeux du patient dans l'obscurité et permettre l'observation par ladite caméra vidéo 3 qui est sensible au rayonnement infrarouge ou proche infrarouge d'observer les mouvements de l'oeil 2 du patient dans l'obscurité de ladite chambre 22.

Conformément à l'invention lesdites caméras 3 et 5 d'axes optiques respectifs XX1 et XX2 qui sont sensiblement confondus et sensiblement parallèles et proches de la direction 29 du regard du patient sont montées "tête bèche" à l'intérieur et/ou sur ledit module 16.

Par référence à la figure 3 dans le cas où l'on souhaite observer les mouvements des yeux du patient avec une bande passante supérieure à 25 hertz qui est la bande passante habituelle des signaux standard vidéo délivrés par des capteurs CCD des caméras habituellement utilisées, il est possible d'équiper la face interne dudit module 16 de photodiodes 30, 31 ou photorécepteurs sensibles à la lumière infrarouge émise par lesdites diodes électroluminescentes 21 ; à cet effet, il est intéressant de prévoir au moins trois photorécepteurs 30, 31, par exemple quatre comme illustré à la figure 3 ; dans ce mode de réalisation, deux photo-récepteurs 30 montés de part et d'autre de ladite caméra 3 sensiblement verticalement superposés, permettent de détecter les mouvements verticaux de l'oeil observé, en digitalisant la tension délivrée par un étage d'analyse haute fréquence dont les entrées différentielles reçoivent les signaux desdites photodiodes ou photorécepteurs.

De la même façon, deux photodiodes ou photorécepteurs 31 situés de part et d'autre de ladite caméra 3 et situés sensiblement sur un même axe horizontal permettent de délivrer une tension sortant de l'étage d'analyse différentielle des signaux issues de ces photorécepteurs.

La face interne du boitier 28 du module 16 illustré à la figure 3 peut également être munie d'une diode électroluminescente 32, émettant de la lumière visible et située également à proximitè de l'objectif de la caméra 3 d'axe optique XX1.

Par référence à la figure 2, le dispositif peut être utilisé pour multiplexer le signal 4 délivré par ladite caméra d'observation de l'oeil (repère 3 de la figure 1) et le signal vidéo 6 délivré par ladite caméra d'observation du décor (repère 5 de la figure 1).

Un premier signal vidéo multiplexé 12 peut être obtenu qui est constitué par exemple par la trame paire du premier signal vidéo 4 et par la trame impaire du deuxième signal vidéo 6 ; à cet effet lesdits signaux vidéo 4 et 6 sont alternativement commutés par deux interrupteurs 26 et 27 qui sont commandés en opposition de phase.

A cet effet, le signal vidéo 6 est présenté à un séparateur 23 de synchro-trame, qui délivre un signal de synchronisaton à une horloge ou monostable 24 dont le signal de sortie sert à commander un interrupteur 26 ; ledit signal de sortie dudit monostable pilote également par l'intermédiaire d'un inverseur 25, ledit deuxième interrupteur 27, de sorte que l'on obtient en sortie un premier signal vidéo multiplexé 12.

Par référence à la figure 4, un deuxième système de multiplexage desdits signaux 4 et 6 vidéo peut être utilisé afin de faciliter et d'accelérer l'analyse desdits signaux vidéo aux fins d'observation du mouvement de l'oeil et des mouvements relatifs de la tête du patient par rapport au décor et/ou du buste du patient par rapport au décor.

A cet effet, lesdits signaux 4 et 6 sont appliqués à l'entrée d'un mélangeur 33 ou multiplexeur, qui reconstitue un deuxième signal vidéo multiplexé 13 qui comporte dans chacune de ses trames paires ou impaires, des lignes issues des trames respectivement paires ou impaires de chacun desdits signaux 4 et 6, ledit multiplexeur 33 etant synchronisé sur l'un desdits signaux par exemple le signal 6, par un dispositif 23 de synchronisation.

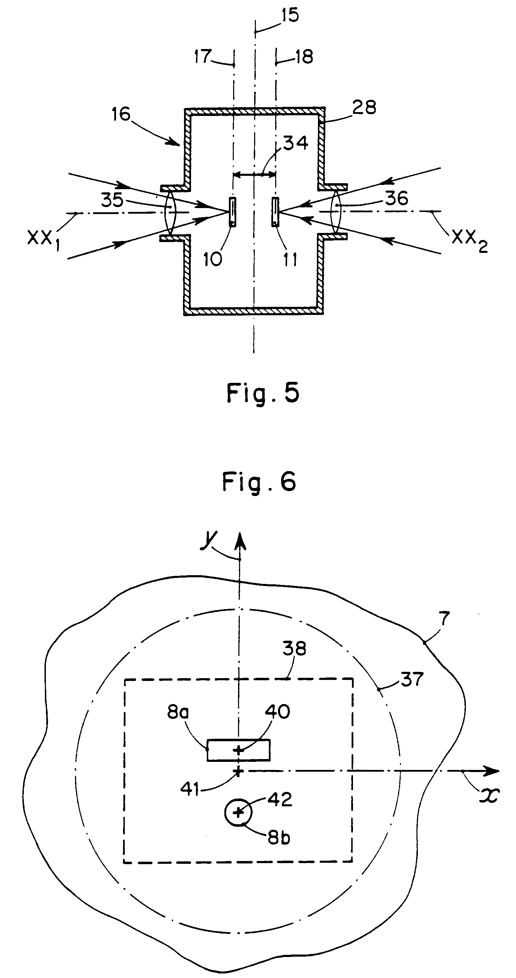

Par référence à la figure 5, ladite caméra vidéo d'observation de l'oeil (repère 3 de la figure 1), comporte essentiellement un capteur matriciel CCD monochrome 10 qui reçoit les rayons lumineux par l'intermédiaire d'une optique ou lentille 35, d'axe optique XX1.

Ladite caméra d'observation du décor intégrée également au boitier 28 du module 16 qui peut être monté de manière amovible sur ledit masque, comporte un capteur CCD matriciel 11 avantageusement identique audit capteur CCD 10, et comporte une optique 36 qui peut être également identique à l'optique 35 de ladite caméra d'observation de l'oeil mis à part le réglage de chaque distance focale pour obtenir les nettetés d'image désirées.

Avantageusement l'optique 36 peut être équipée d'un objectif de très grand angle compris entre 90° et 180° pour permettre aux objets du décor d'être toujours localisés dans l'image malgré par exemple une rotation très importante de la tête du patient.

Avantageusement lesdits capteurs CCD matriciels 11 et 10 sont respectivement situés dans des plans 18, 17 parallèles audit plan de symétrie 15 et situés à égale distance de celui ci, de sorte que la distance 34 entre lesdits capteurs CCD 10 et 11 est faible.

A la figure 6 est illustré schématiquement un décor 7 auquel peut être lié un repère fixe 8a de forme sensiblement rectangulaire et de centre 40.

Sur la figure 6 est schématiquement représentée la limite 37 du champ de vision observé par la caméra de décor du dispositif selon l'invention à l'intérieur duquel peut être prédéfinie une fenêtre 38 d'observation du décor, laquelle fenêtre est dotée d'un centre 41.

Sur cette figure est également illustré un repère 8b de forme par exemple circulaire et de centre 42, lequel repère 8b peut être mobile et constitué par une tâche lumineuse projetée sur ledit décor par un dispositif d'éclairage lié au buste du patient et/ou à la tête et/ou audit module amovible.

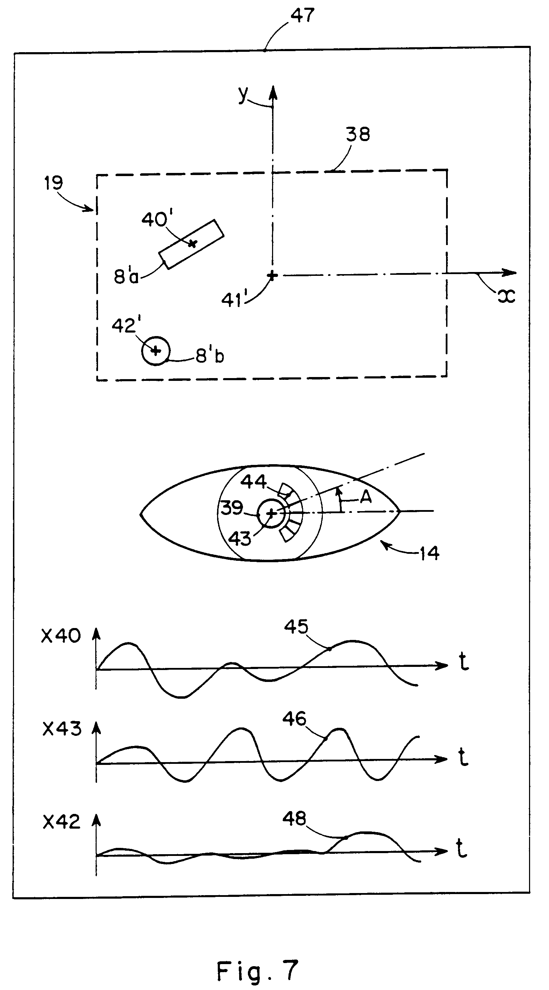

Sur la figure 7 est illustré un exemple de visualisation des résultats de mesure du mouvement de l'oeil et du mouvement de la tête par rapport au décor (par référence à une situation initiale correspondant à la figure 6), utilisant un dispositif selon l'invention.

Les résultats peuvent être visualisés sur un écran 47 faisant partie par exemple d'un micro ordinateur doté de moyens de calcul et de traitement habituels.

On peut visualiser sur ledit écran 47, de préférence simultanément, par exemple à une fréquence de l'ordre de 25 hertz, une image 19 correspondant au décor observé par ladite caméra de décor, une image 14 correspondant à l'image de l'oeil délivrée par ladite caméra dudit module d'observation de l'oeil, ainsi qu'un chronogramme des positions calculées à chaque instant, du barycentre des différents repères et de la pupille de l'oeil.

Dans la partie supérieure de la figure 7 on voit que ladite image 19 du décor peut être limitée par ladite fenêtre 38 prédéterminée et de centre 41' ; dans le plan de la figure sont repérés des axes de coordonnées X et Y passant par ledit centre 41 de ladite fenêtre.

Ledit repère 8a de la figure 6 peut être du fait du mouvement de la tête du patient notamment transformé en un repère 8'a de forme similaire et de centre 40', dont la position par rapport au centre 41' de ladite fenêtre s'est déplacée du fait dudit mouvement de la tête.

De la même façon sur cette image du décor, peut être reconnu un repère 8'b correspondant audit repère 8b de la figure 6 et dont la position du centre 42' par rapport à la position dudit centre 41' de ladite fenêtre 38 s'est également déplacée du fait par exemple d'un mouvement du buste du patient.

Dans la partie médiane de la figure 7 est également illustrée l'image 14 de l'oeil obtenue par ladite caméra d'observation de l'oeil sur laquelle peut être reconnue l'image 39 de la pupille de l'oeil, de centre ou barycentre 43, dont la position dans le plan de la figure peut être détectée et mesurée afin d'analyser les mouvements de l'oeil en réponse à des stimuli.

La variation de la position angulaire A de l'oeil peut être par exemple déterminée par la mesure de la position angulaire d'une strie 44 qui peut être observée dans une couronne située autour de ladite pupille.

Avantageusement, dans un procédé selon l'invention on peut visualiser simultanément les courbes ou chronogrammes 45, 46, 48, correspondant à l'évolution en fonction du temps t, respectivement de l'abscisse X40 dudit repère fixe 8'a par rapport au centre de ladite fenêtre 38 d'observation, de l'abscisse X43 correspondant à la position horizontale du centre de ladite pupille dudit oeil, et de l'abscisse X42 correspondant à la position horizontale dudit repère mobile 8'b lié à la position du torse du patient.

L'invention peut être mise en oeuvre en utilisant par exemple un micro-ordinateur équipé d'une carte d'acquisition de signaux vidéo.

Avantageusement, une telle carte peut comporter une sortie programmable de signaux vidéo sur laquelle peut-être connecté un vidéo projecteur ; on peut ainsi synthétiser des images comportant des repères (cibles ou motifs) qui seront projetés par le vidéo projecteur sur l'écran qu'observe le patient.

Selon ce mode particulier de réalisation de l'invention, on peut utiliser une seule carte électronique pour assurer l'acquisition et la numérisation des signaux délivrés par lesdites premières et deuxièmes caméras et pour assurer la synthèse d'une ou plusieurs images de cible ou repères, ce qui présente un gros avantage économique et de simplicité par rapport à des dispositifs existants permettant de délivrer des cibles dans le champs visuel du patient, et qui sont généralement constitués soit par des stimulateurs d'oculomotricité à barre de diodes électroluminescentes ou comportant un émetteur à faisceau laser, ou bien par des stimulateurs de poursuite réflexe optocinétique à projecteur tournant de type planétarium, boule à mosaïque de miroirs de Zeitoun, ces dispositifs connus étant à la fois très onéreux et très encombrants, par ailleurs dans ce mode de réalisation, la possibilité de création de motifs est illimitée ; de plus, grâce au fait que le même appareil (le micro-ordinateur convenablement programmé) contrôle à la fois l'acquisition et la numérisation des images issus des signaux vidéo délivrés par les caméras et la synthèse des cibles projetées dans le champ de vision du patient,le calcul du mouvement des yeux et/ou de la tête est facilité grâce au fait qu'un même logiciel synthétise lesdits repères de décor et ce même logiciel recherche la forme et calcule la position de ces repères synthétisés pour calculer le mouvement de la tête du patient déduit des signaux délivrés par les caméras ; il est ainsi avantageux lorsque les mouvements de tête s'inscrivent dans une plage angulaire réduite (plus ou moins 60°), de synthétiser par programmation les repères que l'on utilise pour la détection du mouvement plutôt que d'avoir à placer des repères fixes contrastés et lumineux ou très réfléchissant dans le décor, car cela facilite l'installation et l'utilisation du système d'analyse du mouvement de l'oeil.

Selon une caractéristique particulière de l'invention, le dispositif peut comporter un émetteur portable de signaux radioélectriques qui seront obtenus ou élaborés à partir desdits premiers et deuxièmes signaux vidéos délivrés par lesdites première et deuxième caméra, lequel émetteur est porté par le patient, par exemple fixé sur ladite monture.

Ceci peut permettre d'envoyer directement l'image de l'oeil du patient à un récepteur de signaux radioélectriques connecté au microordinateur ou au système informatique de traitement, sans utiliser de câble de liaison, ce qui améliore le confort d'utilisation ; ce procédé d'utilisation est particulièrement intéressant dans le cas où lesdits premier et deuxième signaux vidéo sont multiplexés pour constituer un seul signal vidéo multiplexé, ce qui facilite à l'aide d'un seul ensemble émetteur radio (porté par le patient) et récepteur radio (connecté au calculateur ou ordinateur de traitement), de recevoir sur une seule carte d'acquisition et de numérisation intégré à l'ordinateur, les images de l'oeil et du décor.

## Revendications

1. Dispositif d'analyse du mouvement de l'oeil (2) comportant une première caméra (3) vidéo pouvant délivrer un premier signal (4) vidéo représentatif d'une image (14) dudit oeil, lequel dispositif comporte en outre une deuxième caméra (5) vidéo pouvant délivrer au moins un deuxième signal (6) vidéo représentatif d'une image (19) d'un décor (7), lesquelles première caméra (3) et deuxième caméra (5) sont montées sur une monture (9) de lunette, caractérisé en ce que lesdites première caméra (3) et deuxième caméra (5) sont mécaniquement rigidement liées entre elles et font partie d'un module (16) amovible, et en ce que lesdites première caméra (3) et deuxième caméra (5) ont des points de visée de leurs scènes respectives qui sont rapprochés.

2. Dispositif selon la revendication 1 dans lequel lesdites première caméra (3) et deuxième caméra (5) vidéo dudit module (16) ont des axes optiques (XX1, XX2) respectifs sensiblement confondus et sont montées "tête bêche".

3. Dispositif selon l'une quelconque des revendications 1 à 2. dans lequel ladite monture (9) de lunette est munie par emboîtage d'un deuxième module amovible d'observation et/ou d'analyse de l'oeil comportant une seule caméra, et dans lequel lesdits modules comportent des moyens d'éclairage en lumière infrarouge de l'oeil.

4. Dispositif selon l'une quelconque des revendications 1 à 3, dans lequel ladite monture (9) de lunette est munie de moyens d'étanchéité à la lumière de façon à ce que l'emboîtage de deux modules sur ledit masque permette de former devant lesdits yeux du patient une chambre étanche à la lumière visible extérieure, lequel dispositif comporte un module composé d'un cache.

5. Dispositif selon l'une quelconque des revendications 1 à 4, dans lequel un module amovible comporte une caméra d'observation et/ou d'analyse de l'oeil, comporte des moyens d'éclairage en lumière infrarouge de l'oeil, et comporte des moyens de mesure de l'intensité lumineuse réfléchie par le globe oculaire.

6. Dispositif selon l'une quelconque des revendications 1 à 5 comportant des moyens de numérisation dudit deuxième signal vidéo (6) et des moyens d'analyse d'images de décor numérisées obtenues, lesquels moyens d'analyse comportent des moyens d'extraction de contour et comportent des moyens d'identification d'au moins un objet apparaissant dans ladite image de décor et comportent des moyens de calcul de coordonnées dudit objet.

7. Dispositif selon l'une quelconque des revendications 1 à 6 dans lequel ledit module (16) comporte un premier capteur (10) CCD matriciel et un deuxième capteur (11) CCD matriciel, lesquels premier et deuxième capteurs CCD sont identiques et sont situés dans deux plans (17, 18) sensiblement parallèles entre eux, perpendiculaires auxdits axes optiques sensiblement confondus desdites caméras, et symétriques par rapport audit plan de symétrie (15), lesquels plans (17, 18) et/ou lesquels capteurs CCD sont très proches l'un de l'autre.

8. Dispositif selon l'une quelconque des revendications 1 à 7 comportant des moyens de synchronisation desdits premier et deuxième signaux vidéo (4, 6) et des moyens de multiplexage desdits premier et deuxième signaux vidéo pour former un premier signal vidéo multiplexé composé d'une trame paire et d'une trame impaire, ladite trame paire étant identique à la trame simultanée dudit premier signal vidéo (ou dudit deuxième signal vidéo), et ladite trame impaire étant identique à la trame simultanée dudit deuxième signal vidéo (ou dudit premier signal vidéo respectivement).

9. Dispositif selon l'une quelconque des revendications 1 à 8 dans lequel ledit décor comporte au moins un repère contrasté fixe ou mobile, et ledit dispositif comporte des moyens de projection d'au moins une tache lumineuse constituant ledit repère.

10. Dispositif selon l'une quelconque des revendications 1 à 9 comportant un émetteur portable de signaux radioélectriques obtenus à partir desdits premier et deuxième signaux vidéo.

## Claims

1. Device for analysing the movement of the eye (2), comprising a first video camera (3) capable of delivering a first video signal (4) representative of an image (14) of the said eye, which device further comprises a second video camera (5) capable of delivering at least one second video signal (6) representative of an image (19) of a scene (7), which first camera (3) and second camera (5) are mounted on a spectacle frame (9), characterized in that the said first camera (3) and second camera (5) are mechanically rigidly connected together and form part of a removable module (16), and in that the said first camera (3) and second camera (5) have points of sight of their respective scenes which are close.

2. Device according to Claim 1, in which the said first video camera (3) and second video camera (5) of the said module (16) have substantially merged, respective optical axes (XX1, XX2) and are mounted "head to tail".

3. Device according to either one of Claims 1 and 2, in which the said spectacle frame (9) is provided, by fitting, with a second removable module for observation and/or analysis of the eye, comprising a single camera, and in which the said modules comprise means for lighting the eye with infrared light.

4. Device according to any one of Claims 1 to 3, in which the said spectacle frame (9) is provided with light-tight means so that the fitting of two modules on the said frame makes it possible to form in front of the patient's eyes a chamber which is tight to the outside visible light, which device comprises a module composed of a mask.

5. Device according to any one of Claims 1 to 4, in which a removable module comprises a camera for observing and/or analysing the eye, comprises means for lighting the eye with infrared light, and comprises means for measuring the light intensity reflected by the eyeball.

6. Device according to any one of Claims 1 to 5, comprising means for digitalizing the said second video signal (6) and means for analysing digitalized scene images obtained, which analysing means comprise contour extraction means and comprise means for identifying at least one object appearing in the said scene image and comprise means for calculating coordinates of the said object.

7. Device according to any one of Claims 1 to 6, in which the said module (16) comprises a first matrix CCD sensor (10) and a second matrix CCD sensor (11), which first and second CCD sensors are identical and are located in two planes (17, 18) substantially parallel to each other, perpendicular to the said substantially merged optical axes of the said cameras, and symmetrical with respect to the said plane of symmetry (15), which planes (17, 18) and/or which CCD sensors are very close to one another.

8. Device according to any one of Claims 1 to 7, comprising means for synchronizing the said first and second video signals (4, 6) and means for multiplexing the said first and second video signals to form a first multiplexed video signal composed of an even frame and an odd frame, the said even frame being identical to the simultaneous frame of the said first video signal (or of the said second video signal), and the said odd frame being identical to the simultaneous frame of the said second video signal (or of the said first video signal, respectively).

9. Device according to any one of Claims 1 to 8, in which the said scene comprises at least one fixed or mobile contrasted mark, and the said device comprises means for projecting at least one light spot constituting the said mark.

10. Device according to any one of Claims 1 to 9, comprising a portable emitter of radioelectric signals obtained from the said first and second video signals.

## Patentansprüche

1. Vorrichtung zur Analyse der Bewegung des Auges (2), mit einer ersten Videokamera (3), die ein erstes Videosignal (4) liefern kann, das für ein Bild (14) des Auges repräsentativ ist, wobei die Vorrichtung weiterhin eine zweite Videokamera (5) aufweist, das wenigstens ein zweites Videosignal (6) liefern kann, das repräsentativ für ein Bild (19) einer Umgebung (7) ist, wobei die erste Kamera (3) und die zweite Kamera (5) auf einer Brillenfassung (9) angebracht sind, dadurch gekennzeichnet, daß die erste Kamera (3) und die zweite Kamera (5) mechanisch starr miteinander verbunden sind und einen Teil eines abnehmbaren Moduls (16) bilden und daß die erste Kamera (3) und die zweite Kamera (5) Haltepunkte ihrer jeweiligen Szenen haben, die näher beieinander liegen.

2. Vorrichtung nach Anspruch 1, bei der die erste Kamera (3) und die zweite Videokamera (5) des Moduls (16) jeweilige optischen Achsen (XX1, XX2) haben, die im wesentlichen übereinstimmen, und die "verkehrt zueinander" angebracht sind.

3. Vorrichtung nach einem der Ansprüche 1 bis 2, bei der die Brillenfassung (9) durch Einschieben mit einem zweiten abnehmbaren Modul für die Beobachtung und/oder die Analyse des Auges versehen ist, mit einer einzigen Kamera, und bei der die Module Einrichtungen zum Beleuchten des Auges mit infrarotem Licht aufweisen.

4. Vorrichtung nach einem der Ansprüche 1 bis 3, bei der die Brillenfassung (9) mit Einrichtungen zum Abdichten gegen Licht versehen ist, derart, daß das Einschieben der beiden Module auf die Maske es erlaubt, vor den Augen des Patienten eine Kammer zu bilden, die gegen von außen kommendes sichtbares Licht dicht ist, wobei die Vorrichtung ein Modul aufweist, das aus einer Abdeckung besteht.

5. Vorrichtung nach einem der Ansprüche 1 bis 4, bei der ein abnehmbares Modul eine Kamera zum Beobachten und/oder zur Analyse des Auges aufweist, Einrichtungen zum Beleuchten des Auges mit infrarotem Licht aufweist und Einrichtungen zum Messen der Lichtintensität, die von dem Augapfel reflektiert wird, aufweist.

6. Vorrichtung nach einem der Ansprüche 1 bis 5, mit Einrichtungen zum Digitalisieren des zweiten Videosignals (6) und Einrichtungen zur Analyse von erhaltenen digitalisierten Umgebungsbildern, wobei die Analyseeinrichtungen Einrichtungen zum Herausziehen einer Kontur aufweisen und Einrichtungen für die Identifikation wenigstens eines Gegenstandes, der in dem Umgebungsbild erscheint, aufweisen und Recheneinrichtungen für die Koordinaten des Gegenstandes aufweisen.

7. Vorrichtung nach einem der Ansprüche 1 bis 6, bei der das Modul (16) einen ersten Matrix-CCD-Aufnehmer (10) und einen zweiten Matrix-CCD-Aufnehmer (11) aufweist, wobei der erste und zweite CCD-Aufnehmer identisch sind und in zwei Ebenen (17, 18) liegen, die im wesentlichen parallel zueinander sind, senkrecht zu den im wesentlichen übereinstimmenden optischen Achsen der Kameras und symmetrisch in bezug auf die Symmetrieebene (15), wobei die Ebenen (17, 18) und/oder die CCD-Aufnahme sehr nahe beieinander sind.

8. Vorrichtung nach einem der Ansprüche 1 bis 7, mit Einrichtungen zum Synchronisieren des ersten und zweiten Videosignals (4, 6) und mit Einrichtungen zum Multiplexen des ersten und zweiten Videosignals, um ein erstes gemultiplextes Videosignal zu bilden, das aus einem geraden Teil und aus einem ungeraden Teil besteht, wobei der gerade Teil identisch dem gleichzeitigen Teil des ersten Videosignals (oder des zweiten Videosignals) ist, und der ungerade Teil identisch dem gleichzeitigen Teil des zweiten Videosignals (bzw. des ersten Videosignals ist).

9. Vorrichtung nach einem der Ansprüche 1 bis 8, bei dem die Umgebung wenigstens eine feste oder bewegliche kontrastreichen Markierung aufweist und die Vorrichtung Einrichtungen für die Projektion wenigstens eines Lichtfleckes, die die Markierung bildet, aufweist.

10. Vorrichtung nach einem der Ansprüche 1 bis 9, mit einem tragbaren Sender für funkelektrische Signale, die aus den ersten und zweiten Videosignalen erhalten worden sind.
